# EUROPÄISCHE PATENTSCHRIFT

(11) **EP 0 314 668 B1**
(45) Veröffentlichungstag und Bekanntmachung des Hinweises auf die Patenterteilung: **25.08.1993**
(21) Anmeldenummer: 88890270.7
(22) Anmeldetag: 28.10.1988
(51) Int. Cl.: F16F 9/44, A61F 2/60, G05B 11/60

(54) **Hydraulische Steuerung**
Hydraulic control
Commande hydraulique

(30) Priorität: 30.10.1987 AT 2864/87
(43) Veröffentlichungstag der Anmeldung: 03.05.1989
(73) Patentinhaber: Otto Bock Orthopädische Industrie Besitz- und Verwaltungs-KG, 37115 Duderstadt (DE)
(72) Erfinder: Horvath, Eduard, A-1070 Wien (AT)
(74) Vertreter: Itze, Peter, Dipl.-Ing.

(56) Entgegenhaltungen:
- EP-A- 0 237 085
- WO-A-84/01605
- DE-U- 8 708 011
- GB-A- 2 194 309
- US-A- 2 859 451

## Beschreibung

Die Erfindung bezieht sich auf eine Steuerschaltung für die Wirkung eines hydraulischen, durch eine Kolben/Zylindereinheit gebildeten Reglers, insbesondere für die Regelung der Bewegung künstlicher Gelenke an Menschen, bei welchem Regler die an beiden Seiten des Kolbens befindlichen Zylinderräume durch einen ein Regelorgan für den Durchfluß von der die Kolbenstange aufweisenden Kolbenseite zur gegenüberliegenden Kolbenseite aufweisenden Durchgang verbunden sind, wobei zudem beide Zylinderräume mit einem Kompensationsraum zum Ausgleich der aufgrund der Kolbenstange unterschiedlichen Volumina der Zylinderräume in Verbindung stehen, wobei in der Verbindungsleitung zwischen dem von der Kolbenstange freien Zylinderraum und dem Kompensationsraum ein das Durchströmen in Richtung zum Kompensationsraum verhinderndes Rückschlagventil vorgesehen ist und wobei der die Kolbenstange aufweisende Zylinderraum über eine, ein weiteres Rückschlagventil aufweisende Leitung füllbar ist. Eine derartige Ausbildung ist aus der WO-A-84/01605 bekannt. Bei dieser bekannten Ausbildung handelt es sich um einen Schwingungsdämpfer für Sitze, insbesondere Fahrzeugsitze. Dabei sind zwei Verbindungskanäle zwischen den beiden Zylinderräumen vorgesehen, wobei in jedem derselben ein eigenes Steuerventil für den Flüssigkeitsdurchtritt eingeschaltet ist. Eine solche Ausbildung ist sehr aufwendig und benötigt entsprechenden Platz.

Bei einer anderen bekannten Ausbildung ist das erste einstellbare Drosselorgan im Strömungsverlauf von dem von der Kolbenstange freien Zylinderraum zum Kompensationsraum angeordnet. Dadurch kann mit diesem einstellbaren Drosselorgan lediglich die Bewegung der Kolbenstange in Richtung eines Verkürzens der Kolben/Zylindereinheit gesteuert werden, wogegen die Rückbewegung des Kolbens bestenfalls durch einen zwischen dem die Kolbenstange aufweisenden Zylinderraum und dem Kompensationsraum vorgesehenen Drosselspalt gedrosselt werden kann. Keinesfalls ist jedenfalls die Rückbewegung, also das Strecken der Kolben- und Zylindereinheit, geregelt steuerbar.

Es hat sich nun gezeigt, daß insbesondere für die Regelung der Bewegung künstlicher Gelenke an Menschen es angezeigt ist, die Streckbewegung der Gelenke, also das Ausziehen der Kolben/Zylindereinheit geregelt steuern zu können, da nur dadurch ein möglichst natürlicher Bewegungsablauf der Gelenke erreicht wird.

Der Erfindung liegt die Aufgabe zugrunde, eine Steuerschaltung der eingangs genannten Art zu schaffen, mit welcher eine Regelung beider Bewegungsrichtungen des Kolbens im Zylinder bewirkt werden kann.

Erfindungsgemäß wird diese Aufgabe dadurch gelöst, daß das im Strömungsverlauf von dem von der Kolbenstange freien Zylinderraum zum Kompensationsraum ein erstes einstellbares Drosselorgan vorgesehen ist, das in einer, das Rückschlagventil umgehenden Bypaßleitung angeordnet ist, wobei im Strömungsverlauf von dem die Kolbenstange aufweisenden Zylinderraum zum Kompensationsraum ein weiteres einstellbares Drosselorgan angeordnet ist. Durch eine solche Ausbildung wird erreicht, daß es genügt, in den Verbindungsleitungen zwischen den Zylinderräumen und dem Kompensationsraum Rückschlagventile anzuordnen, um den gewünschten Effekt zu erreichen. Diese Rückschlagventile beeinflussen die Strömung in der nicht gesperrten Richtung nicht, was eine störungsfreie Füllung des unbeaufschlagten Kolbenraumes ergibt, wobei der beaufschlagte Kolbenraum die verdrängte Flüssigkeit über das zugeordnete einstellbare Drosselorgan abführt. Es wird dabei bei einer Ausziehbewegung des Kolbens die aus dem mit der Kolbenstange versehenen Kolbenraum verdrängte Flüssigkeit über das weitere Drosselorgan in den Kompensationsraum gepreßt, wodurch durch dieses Drosselorgan die Ausziehbewegung regelbar ist. Bei der Einschubbewegung des Kolbens in den Zylinder kann die verdrängte Hydraulikflüssigkeit zur Gänze über das erste einstellbare Drosselorgan in den Kompensationsraum gepreßt werden, wobei jene Flüssigkeitsmenge, welche zur Füllung des hinter dem Kolben liegenden Zylinderraums benötigt wird, von dem Kompensationsraum direkt in diesen Kolbenraum eingebracht wird. Da jedoch bei der Rückbewegung in der Regel eine andere Regelung gewünscht wird, ist das in der Bypaßleitung vorgesehene erste einstellbare Drosselorgan so eingestellt, daß der durchströmenden Flüssigkeit ein anderer Widerstand entgegengesetzt wird als durch das weitere einstellbare Drosselorgan. Damit ist eine Ausführung erzielt, bei welcher die Hauptbewegungsrichtung die Ausziehbewegung des Kolbens aus dem Zylinder ist, und bei welcher aber auch die Einschiebebewegung des Kolbens in den Zylinder mittels eines einstellbaren Drosselorganes steuerbar ist.

Vorteilhafterweise kann das weitere Rückschlagventil im Durchgang zwischen den beiden Kolbenräumen angeordnet sein. Dadurch wird erreicht, daß bei der Kolbenbewegung im Sinne des Einschiebens jener Teil der Hydraulikflüssigkeit, der zur Füllung des die Kolbenstange aufweisenden Kolbenraumes benötigt wird, direkt über den Durchgang gefördert wird. Bei einer besonders vorteilhaften Variante kann das zwischen beiden Zylinderräumen vorgesehene Rückschlagventil als Wechselventil ausgebildet sein, welches von dem von der Kolbenstange freien Zylinderraum her von der einen Seite beaufschlagt ist, von welcher Leitung die Bypaßleitung zum ersten einstellbaren Drosselorgan ausgeht, wobei von der anderen Seite des Wechselventils die zum weiteren einstellbaren Drosselorgan führende Leitung ausgeht, und wobei von der Mitte des Wechselventils die zu dem die Kolbenstange aufweisenden Zylinderraum führende Leitung ausgeht. Durch ein solches Wechselventil ist es möglich, bei der Einschubbewegung des Kolbens in den Zylinder das weitere einstellbare Drosselorgan aus dem Flüssigkeitsweg auszuschalten, sodaß die gesamte überschüssige, der Differenz des veränderten Zylinderraums entsprechende Flüssigkeitsverdrängung über das erste einstellbare Drosselorgan geführt wird, wodurch eine völlig unabhängige Steuerung der Dämpfung der Kolben/Zylindereinheit in beiden Bewegungsrichtungen des Kolbens innerhalb des Zylinders erzielt ist. Um eine noch bessere Anpassung der Regelungswirkung an Bewegungen künstlicher Gelenke zu erzielen, können zu den beiden einstellbaren Drosselorganen weitere, bei Ansteigen des Druckes schließende Drosselorgane parallelgeschaltet sein, wobei gegebenenfalls noch weitere Drosselorgane, die bei noch weiterem Ansteigen des Druckes öffnen, vorgesehen sind. Durch die bei Ansteigen des Druckes schließenden Drosselorgane wird erreicht, daß trotz Ansteigen des Druckes auf der einen Kolbenseite die Kolbenbewegung innerhalb des Zylinders nicht beschleunigt wird, sodaß bei einer aufgrund der Bewegung anderen Hebelwirkung des künstlichen Gelenks des Menschen keine schnellere Bewegung der einzelnen durch das künstliche Gelenk verbundenen Teile erfolgt. Um allerdings bei zu starkem Ansteigen des Druckes eine Zerstörung einzelner Teile der Steuerung zu vermeiden, können, wie schon angeführt, die noch weiteren Drosselorgane vorgesehen sein. In besonders vorteilhafter Ausbildung können die weiteren, bei Ansteigen des Druckes schließenden bzw. die bei noch weiterem Ansteigen des Druckes öffnenden Drosselorgane durch Federbelastung rückstellbar sein.

Bei einer weiteren Ausführungsvariante kann das Regelorgan im Durchgang als Drossel ausgebildet sein, wobei das weitere einstellbare Drosselorgan durch eine Bypaßleitung umgehbar ist, in welcher ein den Durchfluß von dem die Kolbenstange aufweisenden Zylinderraum zum Kompensationsraum sperrendes Rückschlagventil angeordnet ist. Durch eine solche Ausbildung wird erreicht, daß bei ungleichmäßigen Bewegungseigenschaften bzw. Steuerungswirkungen der einstellbaren Drosselorgane fühlbar ungleichmäßige Bewegungseigenschaften ausgeschaltet werden, da über das im Durchgang eingeschaltete Drosselorgan immer ein gewisser Durchfluß von Flüssigkeit von der einen Kolbenseite zur anderen Kolbenseite gegeben ist, wobei zufolge der zwischen den Kolbenräumen und dem Kompensationsraum angeordneten Rückschlagventile eine zuverlässige Füllung des jeweils in Bewegungsrichtung des Kolbens hinten liegenden Zylinderraumes sichergestellt ist, u.zw. ohne daß in diesem Zylinderraum ein höherer Unterdruck auftritt, welcher unter Umständen zu Dampfblasenbildungen in der Flüssigkeit führen könnte.

Bei einer weiteren Ausführungsvariante kann das weitere Rückschlagventil direkt in der Verbindungsleitung zwischen dem die Kolbenstange aufweisenden Zylinderraum und dem Kompensationsraum angeordnet sein, wobei das weitere einstellbare Drosselorgan in einer das Rückschlagventil umgehenden Bypaßleitung liegt. Dadurch wird erreicht, daß die gesamte verdrängte Flüssigkeit über jeweils ein einstellbares Drosselorgan und die zur Füllung des unbeaufschlagten Kolbenraumes benötigte Flüssigkeit direkt aus dem Kompensationsraum angesaugt wird, wodurch verhindert ist, daß in dem unbeaufschlagten Kolbenraum bzw. in der vorgeschalteten Leitung ein Unterdruck entsteht, welcher die Füllung unangenehm beeinflussen könnte. Weiters ist vermieden, daß die Befüllung des jeweils unbeaufschlagten Kolbenraumes über irgendwelche Drosselorgane erfolgt, die gleichfalls z.B. durch Wirbelbildung die Befüllung stören könnten.

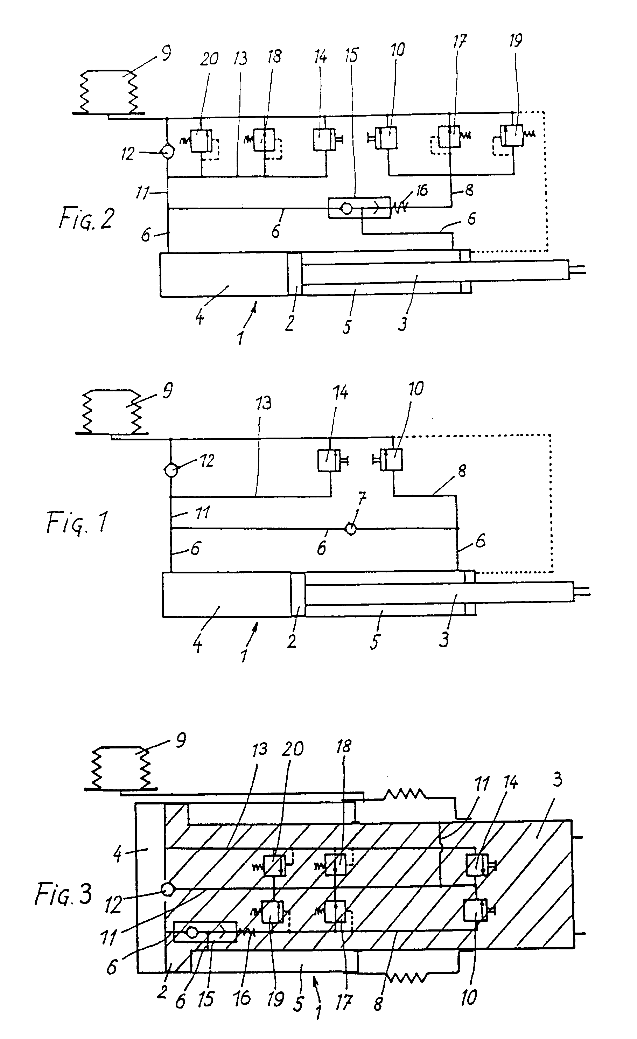

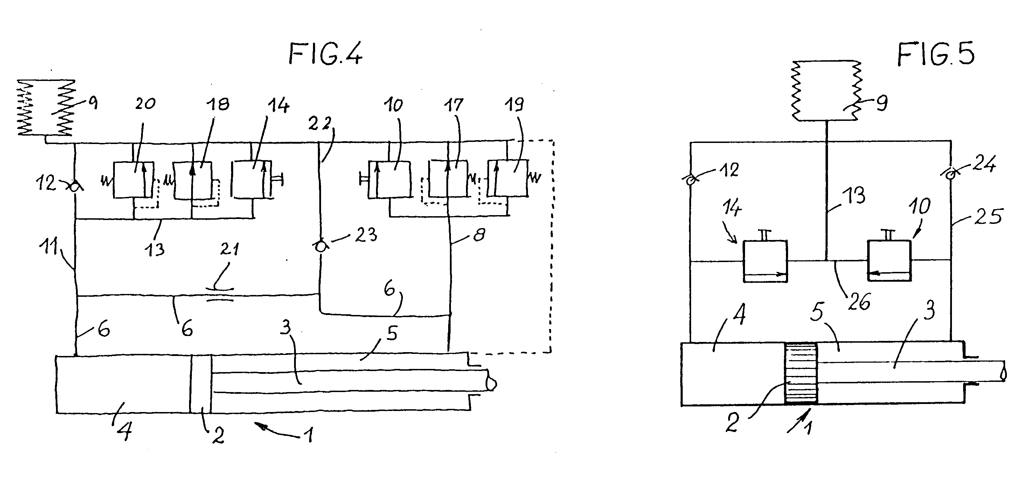

In der Zeichnung sind verschiedene Ausführungsformen der Steuerschaltung in Form schematischer Schaltbilder wiedergegeben. Fig. 1 zeigt die einfachste Ausgestaltung der erfindungsgemäßen Steuerschaltung. Fig. 2 gibt die Schaltung bei vorgesehenen weiteren einstellbaren Drosselorganen wieder. Fig. 3 veranschaulicht eine kompakte Bauform der erfindungsgemäßen Steuerschaltung innerhalb des Kolbens der Kolben/Zylindereinheit. Fig. 4 stellt eine weitere Ausführungsvariante dar, und zwar bei einer der Fig. 2 analogen Drosselorganschaltung. Fig. 5 veranschaulicht schließlich eine sehr einfache Ausführungsform der erfindungsgemäßen Steuerschaltung.

Mit 1 ist ein Zylinder bezeichnet, in welchem ein Kolben 2 mittels einer Kolbenstange 3 in axialer Richtung verschiebbar ist. Mit 4 ist der von der Kolbenstange freie Zylinderraum bezeichnet und mit 5 der durch die Kolbenstange verkleinerte Zylinderraum. Von dem von der Kolbenstange freien Zylinderraum 4 führt eine zum anderen Kolbenraum 5 führende Verbindungsleitung 6 weg, in welcher ein erstes Rückschlagventil eingebaut ist, welches ein Rückfließen der Hydraulikflüssigkeit vom Zylinderraum 5 in den Zylinderraum 4 verhindert. Von der Verbindungsleitung 6 führt eine Leitung 8 zu einem Kompensationsraum 9, welcher zum Ausgleich der unterschiedlichen Volumina der beiden Zylinderräume 4 und 5 dient. In der Leitung 8 ist ein weiteres regelbares Drosselorgan 10 vorgesehen, dessen Drosselwirkung mittels einer nicht dargestellten Einstellschraube steuerbar ist.

Vom Zylinderraum 4 bzw. von der Verbindungsleitung 6 zweigt eine weitere zum Kompensationsraum 9 führende Leitung 11 ab, in welcher ein weiteres Rückschlagventil 12, welches den direkten Fluß der Hydraulikflüssigkeit vom Zylinderraum 4 in den Kompensationsraum 9 abschließt, angeordnet ist. Dieses weitere Rückschlagventil ist über eine Bypaßleitung 13 umgehbar, in welcher ein erstes regelbares Drosselorgan 14 angeordnet ist.

Bei den Ausführungsbeispielen gemäß Fig. 2 und 3 ist das erste Rückschlagventil 7 durch ein Wechselventil 15 ersetzt, welches als doppelt wirkendes Rückschlagventil ausgebildet ist. In dieses Wechselventil 15 mündet die Verbindungsleitung 6 an dem einen Ende ein, und führt zwischen den beiden Ventilsitzen des Wechselventils zum anderen Zylinderraum zurück. Am anderen Ende des Wechselventils führt die Leitung 8 zum ersten regelbaren Drosselorgan weg. Das Wechselventil ist dabei durch eine Feder 16 derart vorbelastet, daß es die in der Zeichnung wiedergegebene, den Rückfluß vom Zylinderraum 5 in den Zylinderraum 4 verhindernde Stellung einnimmt.

Sowohl in der Leitung 8 als auch in der Bypaßleitung 13 sind parallel zum weiteren regelbaren Drosselorgan 10 bzw. zum ersten regelbaren Drosselorgan 14 je ein bei Ansteigen des Druckes schließendes Drosselorgan 17 und 18 sowie je ein bei noch weiterem Ansteigen des Druckes öffnendes Drosselorgan 19 und 20 eingeschaltet.

Wird der Kolben 2 über die Kolbenstange 3 im Zylinder 1 in Richtung eines Herausbewegens des Kolbens aus dem Zylinder, also in den Figuren nach rechts, bewegt, dann wird die im Zylinderraum 5 befindliche Hydraulikflüssigkeit über einen Teil der Verbindungsleitung 6, die Leitung 8 und das weitere regelbare Drosselorgan 10 zum Kompensationsraum 9 gedrängt. Gleichzeitig wird aus dem Kompensationsraum 9 über die Leitung 11, das Rückschlagventil 12 und bei den Fig. 1 und 2 auch noch über einen Teil der Verbindungsleitung 6 Hydraulikflüssigkeit aus dem Kompensationsraum in den Zylinderraum 4 eingesaugt. Da in der Leitung 11 keinerlei Drosselung erfolgt, erfolgt die gesamte Regelung in dieser Bewegungsrichtung über das weitere regelbare Drosselorgan 10.

Wird nun der Kolben 2 in entgegengesetzter Richtung im Zylinder 1 bewegt, also in den Fig. 1 - 3 nach links, dann wird die im Zylinderraum 4 befindliche Hydraulikflüssigkeit über die Verbindungsleitung 6 und das Rückschlagventil 7 zum einen Teil in den Zylinderraum 5 geleitet, wobei gemäß Fig. 1 das dem Differenzvolumen zwischen den beiden Zylinderräumen entsprechende Flüssigkeitsvolumen über die Leitung 8 und das einstellbare Drosselorgan 10 in den Kompensationsraum 9 geleitet wird. Ist das erste einstellbare Drosselorgan 14 in der Bypaßleitung 13 so eingestellt, daß es dem Fluß der Hydraulikflüssigkeit einen geringeren Widerstand entgegensetzt als das weitere regelbare Drosselorgan, dann wird das dem Differenzvolumen entsprechende Flüssigkeitsvolumen über die Bypaßleitung 13 und das erste regelbare Drosselorgan 14 in den Kompensationsraum 9 gelangen.

Bei der Ausführungsform gemäß den Fig. 2 und 3 ist aufgrund des Wechselventils 15, welches bei dieser Kolbenbewegung eine Stellung einnimmt, daß das Abschlußorgan des Wechselventils das Eintreten von Hydraulikflüssigkeit in die Leitung 8 verhindert, das weitere regelbare Drosselorgan 10 vom Durchfluß der Hydraulikflüssigkeit ausgeschaltet. Es wird damit das gesamte dem Differenzvolumen entsprechende Flüssigkeitsvolumen über die Bypaßleitung 13 und das erste regelbare Drosselorgan 14 geleitet, wodurch eine völlig unabhängige Regelung in beiden Kolbenbewegungsrichtungen erreicht ist.

Übersteigt bei den Ausführungsformen nach den Fig. 2 und 3 der in der Leitung 8 bzw. in der Bypaßleitung 13 herrschende Druck einen vorgegebenen Wert, dann schließen die Drosselorgane 17 bzw. 18 den Durchfluß weiter, sodaß der jeweiligen Kolbenbewegung stärker entgegengewirkt wird. Bei weiterem Ansteigen des Druckes in der Leitung 8 bzw. der Bypaßleitung 13 öffnen die Ventile 19 und 20, wodurch einem Blockieren der Gelenke durch zu hohen Gegendruck und damit die Sicherheit der Benützer erhöht wird. Die Drosselorgane 17, 18,19, 20 sind dabei mittels einer Feder in die Ausgangsstellung rückstellbar, wenn die Druckverhältnisse in der Leitung 8 bzw. der Bypaßleitung 13 wieder auf den Normalwert zurückfallen.

Beim Ausführungsbeispiel nach Fig. 4 ist in der Verbindungsleitung 6 zwischen dem Zylinderraum 4 und dem Zylinderraum 5 ein, eine vorgegebene Durchgangsweite aufweisendes Drosselorgan 21 vorgesehen, wobei in dieser Verbindungsleitung in Abwandlung der übrigen Ausführungsbeispiele kein Rückschlagventil angeordnet ist. Vielmehr ist eine das weitere einstellbare Drosselorgan 10 umgehende Bypaßleitung 22 vorgesehen, in welcher ein Rückschlagventil 23 angeordnet ist, welches einen Durchfluß von Flüssigkeit vom Zylinderraum 5 zum Kompensationsraum 9 sperrt.

Wird der Kolben 2 im Zylinder 1 im Sinne eines Verlängerns der Kolben/Zylindereinheit, also in Fig. 1 nach rechts, bewegt, dann wird die aus dem Zylinderraum 5 verdrängte Flüssigkeit dann, wenn der Durchströmwiderstand in der Drossel 21 höher ist als in dem einstellbaren Regelventil, über das Drosselorgan 10 zum Kompensationsraum 9 geführt. In den Zylinderraum 4 wird aus dem Kompensationsraum Flüssigkeit über das sich öffnende Rückschlagventil 12 und die Leitung 11 bzw. einen Teil der Leitung 6 eingesaugt. Es erfolgt damit also die Dämpfung der Bewegung durch das einstellbare Drosselorgan 10. Ist hingegen der Durchströmwiderstand im weiteren einstellbaren Drosselorgan 10 höher als in der Drossel 21, dann wird die aus dem Zylinderraum 5 verdrängte Flüssigkeit über die Verbindungsleitung 6 und die Drossel 21 in den Zylinderraum 4 gepreßt, sodaß aus dem Kompensationsraum 9 über die Leitung 11 und das Rückschlagventil 12 lediglich das Differenzvolumen der zur Füllung des Zylinderraumes 4 benötigten Flüssigkeit angesaugt wird.

Wird nun der Kolben 2 im Sinne eines Einschiebens in den Zylinder 1, also in Fig. 4 gesehen nach links, bewegt, dann wird das für die Füllung des Zylinderraumes 5 benötigte Flüssigkeitsvolumen in Abhängigkeit vom dem der Flüssigkeit entgengesetzten Durchströmwiderstand in der Drossel 21 bzw. im einstellbaren Drosselorgan 14 entweder über die Verbindungsleitung 6 oder über die Bypaßleitung 22 und das Rückschlagventil 23 angesaugt. Das Differenzvolumen zwischen dem aus dem Zylinderraum 4 verdrängten Flüssigkeitsvolumen und dem für die Füllung des Zylinderraumes 5 benötigten Flüssigkeitsvolumen wird über die Leitung 13 und das erste einstellbare Drosselorgan 14 dem Kompensationsraum 9 zugeführt. Durch die Drossel 21 wird auf jeden Fall erreicht, daß ein gewisser Flüssigkeitsdurchtritt von einem Zylinderraum zum anderen Zylinderraum mit einer gleichförmigen Dämpfungswirkung durchtreten kann, sodaß bei unterschiedlich stark eingestellten Drosselorganen 10 bzw. 14 abrupte Übergänge zwischen der Steuerung der Einwärtsbewegung und der Auswärtsbewegung des Kolbens im Zylinder und umgekehrt vermieden sind.

Bei der in Fig. 5 wiedergegebenen Ausführungsvariante handelt es sich um eine besonders einfache Form, bei welcher ein weiteres Rückschlagventil 24 direkt in der Verbindungsleitung 25 zwischen dem die Kolbenstange 3 aufweisenden Zylinderraum 5 und dem Kompensationsraum 9 angeordnet ist, wobei das weitere einstellbare Drosselorgan 10 in einer das Rückschlagventil 24 umgehenden Bypaßleitung 26 liegt.

Wird bei der Ausführungsform nach Fig. 5 der Kolben 2 in den Zylinder 1 hineingeschoben, also in Fig. 5 gesehen nach links bewegt, dann wird die aus dem Zylinderraum 4 verdrängte Flüssigkeit über das erste einstellbare Drosselorgan 14 und die Leitung 13 in den Kompensationsraum 9 gepreßt, da ein anderer Strömungsverlauf durch das Rückschlagventil 12 unterbunden ist. Das Befüllen des an der Rückseite des Kolbens liegenden Zylinderraumes 5 erfolgt unbehindert über die Leitung 25 und das sich öffnende Rückschlagventil 24 aus dem Kompensationsraum 9. Wird bei dem in Fig. 5 wiedergegebenen Ausführungsbeispiel der Kolben aus dem Zylinder herausgezogen, also in Fig. 5 gesehen nach rechts, bewegt, dann wird die aus dem Zylinderraum 5 verdrängte Hydraulikflüssigkeit über das weitere einstellbare Drosselorgan 10 und die Leitungen 26 und 13 in den Kompensationsraum 9 gefördert, wobei die Füllung des Zylinderraumes 4 über die das Rückschlagventil 12 aufweisende Leitung erfolgt, wobei gleichfalls in dieser Leitung außer dem Rückschlagventil kein anderes Regelventil angeordnet ist, welches gegebenenfalls eine Füllung des Zylinderraumes nachteilig beeinflussen könnte.

## Patentansprüche

1. Steuerschaltung für die Wirkung eines hydraulischen, durch eine Kolben/Zylindereinheit gebildeten Reglers, insbesondere für die Regelung der Bewegung künstlicher Gelenke an Menschen, bei welchem Regler die an beiden Seiten des Kolbens (2) befindlichen Zylinderräume (4, 5) durch einen ein Regelorgan für den Durchfluß von der die Kolbenstange (3) aufweisenden Kolbenseite zur gegenüberliegenden Kolbenseite aufweisenden Durchgang (6) verbunden sind, wobei zudem beide Zylinderräume (4, 5) mit einem Kompensationsraum (9) zum Ausgleich der aufgrund der Kolbenstange (3) unterschiedlichen Volumina der Zylinderräume (4, 5) in Verbindung stehen, wobei in der Verbindungsleitung zwischen dem von der Kolbenstange (3) freien Zylinderraum (4) und dem Kompensationsraum (9) ein das Durchströmen in Richtung zum Kompensationsraum (9) verhinderndes Rückschlagventil (12) vorgesehen ist und wobei der die Kolbenstange (3) aufweisende Zylinderraum (5) über eine, ein weiteres Rückschlagventil (23;24) aufweisende Leitung (8;25) füllbar ist, dadurch gekennzeichnet, daß im Strömungsverlauf von dem von der Kolbenstange freien Zylinderraum (4) zum Kompensationsraum (9) ein erstes einstellbares Drosselorgan (14) vorgesehen ist, das in einer, das Rückschlagventil (12) umgehenden Bypaßleitung (13) angeordnet ist, wobei im Strömungsverlauf von dem die Kolbenstange (3) aufweisenden Zylinderraum (5) zum Kompensationsraum (9) ein weiteres einstellbares Drosselorgan (10) angeordnet ist.

2. Steuerschaltung nach Anspruch 1, dadurch gekennzeichnet, daß das weitere Rückschlagventil (7) im Durchgang (6) zwischen den beiden Zylinderräumen (4, 5) angeordnet ist (Fig. 1).

3. Steuerschaltung nach Anspruch 2, dadurch gekennzeichnet, daß das zwischen den beiden Zylinderräumen (4,5) vorgesehene Rückschlagventil als Wechselventil (15) ausgebildet ist, welches von dem von der Kolbenstange (3) freien Zylinderraum (4) her von der einen Seite beaufschlagt ist, von welcher Leitung (6) die Bypaßleitung (13) zum ersten einstellbaren Drosselorgan (14) ausgeht, wobei von der anderen Seite des Wechselventiles (15) die zum weiteren einstellbaren Drosselorgan (10) führende Leitung (8) ausgeht, und wobei von der Mitte des Wechselventiles (15) die zu dem die Kolbenstange (3) aufweisenden Zylinderraum (5) führende Leitung (6) ausgeht (Fig. 2 und 3).

4. Steuerschaltung nach einem der Ansprüche 1 bis 3, dadurch gekennzeichnet, daß zu den beiden einstellbaren Drosselorganen (10,14) weitere bei Ansteigen des Druckes schließende Drosselorgane (17,18) parallel geschaltet sind, wobei gegebenenfalls noch weitere Drosselorgane (19,20) die bei noch weiterem Ansteigen des Druckes öffnen, vorgesehen sind.

5. Steuerschaltung nach einem der Ansprüche 1 bis 4, dadurch gekennzeichnet, daß die weiteren, bei Ansteigen des Druckes schließenden bzw. die bei noch weiterem Ansteigen des Druckes öffnenden Drosselorgane (17,18,19,20) durch Federbelastung rückstellbar sind.

6. Steuerschaltung nach einem der Ansprüche 1, 4 oder 5, dadurch gekennzeichnet, daß das Regelorgan im Durchgang (6) als Drossel (21) ausgebildet ist, wobei das weitere einstellbare Drosselorgan (10), durch eine Bypaßleitung (22) umgehbar ist, in welcher ein den Durchfluß von dem die Kolbenstange (3) aufweisenden Zylinderraum (5) zum Kompensationsraum (9) sperrendes Rückschlagventil (23) angeordnet ist (Fig. 4).

7. Steuerschaltung nach Anspruch 1, dadurch gekennzeichnet, daß das weitere Rückschlagventil (24) direkt in der Verbindungsleitung (25) zwischen dem die Kolbenstange (3) aufweisenden Zylinderraum (5) und dem Kompensationsraum (9) angeordnet ist, wobei das weitere einstellbare Drosselorgan (10) in einer, das Rückschlagventil (24) umgehenden Bypaßleitung (26) liegt (Fig. 5).

## Claims

1. Control circuit for the action of a hydraulic regulator formed by a piston/cylinder unit, in particular for regulating the movement of artificial joints in humans, in which regulator the cylinder chambers (4, 5) located on both sides of the piston (2) are connected by a passage (6) exhibiting a control element for the through-flow from the piston side exhibiting the piston rod (3) to the opposite piston side, both cylinder chambers (4, 5) being additionally linked to a compensation chamber (9) for equalising the cubic capacities of the cylinder chambers (4, 5), which cubic capacities are different due to the piston rod (3), a nonreturn valve (12) preventing through-current in the direction of the compensation chamber (9) being provided in the connecting conduit between the cylinder chamber (4) free from the piston rod (3) and the compensation chamber (9), and the cylinder chamber (5) exhibiting the piston rod (3) being fillable via a conduit (8; 25) exhibiting a further nonreturn valve (23; 24), characterised in that in the current path running from the cylinder chamber (4) free from the piston rod to the compensation chamber (9) there is provided a first adjustable choke element (14), which is arranged in a bypass conduit (13) circumventing the nonreturn valve (12), a further adjustable choke element (10) being arranged in the current path running from the cylinder chamber (5) exhibiting the piston rod (3) to the compensation chamber (9).

2. Control circuit according to Claim 1, characterised in that the further nonreturn valve (7) is arranged in the passage (6) between the two cylinder chambers (4, 5). (Fig. 1)

3. Control circuit according to Claim 2, characterised in that the nonreturn valve provided between the two cylinder chambers (4, 5) is configured as a shuttle valve (15), which, from the cylinder chamber (4) free from the piston rod (3), is acted upon from the one side, from which conduit (6) the bypass conduit (13) to the first adjustable choke element (14) originates, the conduit (8) leading to the further adjustable choke element (10) originating from the other side of the shuttle valve (15), and the conduit (6) leading to the cylinder chamber (5) exhibiting the piston rod (3) originating from the centre of the shuttle valve (15). (Figs. 2 and 3)

4. Control circuit according to one of Claims 1 to 3, characterised in that connected in parallel to the two adjustable choke elements (10, 14) are further choke elements (17, 18) which close in the event of a rise in pressure, there being provided, where appropriate, yet further choke elements (19, 20) which open in the event of a still further rise in pressure.

5. Control circuit according to one of Claims 1 to 4, characterised in that the further choke elements (17, 18, 19, 20) which close in the event of a rise in pressure or which open in the event of a still further rise in pressure are resettable by a spring-loading mechanism.

6. Control circuit according to one of Claims 1, 4 or 5, characterised in that the regulating element in the passage (6) is configured as a choke (21), the further adjustable choke element (10) being able to be circumvented by a bypass conduit (22), in which there is arranged a nonreturn valve (23) shutting off the throughflow from the cylinder chamber (5) exhibiting the piston rod (3) to the compensation chamber (9). (Fig. 4)

7. Control circuit according to Claim 1, characterised in that the further nonreturn valve (24) is arranged directly in the connecting conduit (25) between the cylinder chamber (5) exhibiting the piston rod (3) and the compensation chamber (9), the further adjustable throttle element (10) being situated in a bypass conduit (26) circumventing the nonreturn valve (24). (Fig. 5)

## Revendications

1. Circuit de commande pour le fonctionnement d'un régulateur hydraulique formé par une unité cylindre-piston, en particulier pour la régulation du mouvement de membres artificiels sur des personnes, dans lequel régulateur les chambres de cylindre (4, 5) se trouvant de part et d'autre du piston (2) sont reliées par un passage (6) présentant un organe de régulation pour l'écoulement entre le côté du piston contenant la tige de piston (3) et le côté du piston opposé, les deux chambres de cylindre (4, 5) étant, en outre, reliées à une chambre de compensation (9) pour équilibrer les différences de volume des chambres de cylindre (4, 5), dues à la tige de piston (3), une soupape de retenue (12) étant prévue dans la conduite de liaison entre la chambre du cylindre (4) dépourvue de tige de piston (3) et la chambre de compensation (9), qui empêche l'écoulement en direction de la chambre de compensation (9), et la chambre de cylindre (5) contenant la tige de piston (3) pouvant être remplie par l'intermédiaire d'une conduite (8 ; 25) présentant une autre soupape de retenue (23 ; 24), caractérisé en ce qu'un premier organe d'étranglement (14) réglable est prévu dans le parcours d'écoulement entre la chambre de cylindre (4) dépourvue de tige de piston et la chambre de compensation (9), et est disposé dans une conduite de dérivation (13) contournant la soupape de retenue (12), un autre organe d'étranglement (10) réglable étant disposé dans le parcours d'écoulement entre la chambre de cylindre (5) contenant la tige de piston (3) et la chambre de compensation (9).

2. Circuit de commande selon la revendication 1, caractérisé en ce que l'autre soupape de retenue (7) est disposée dans le passage (6) entre les deux chambres de cylindre (4, 5) (Fig. 1).

3. Circuit de commande selon la revendication 2, caractérisé en ce que la soupape de retenue prévue entre les deux chambres de cylindre (4, 5) est conformée en soupape à deux voies (15), alimentée par un côté, depuis la chambre de cylindre (4) dépourvue de tige de piston (3), la conduite de dérivation (13) en partant de la conduite (6) pour aller vers le premier organe d'étranglement (14) réglable, la conduite (8) conduisant à l'autre organe d'étranglement (10) réglable partant de l'autre côté de la soupape à deux voies (15), et la conduite (6) conduisant à la chambre de cylindre (5) contenant la tige de piston (3) en partant du milieu de la soupape à deux voies (15) (Fig. 2 et 3).

4. Circuit de commande selon l'une des revendications 1 à 3, caractérisé en ce qu'en plus des deux organes d'étranglement (10, 14) réglables, d'autres organes d'étranglement (17, 18) sont disposés en parallèle, qui se ferment lorsque la pression monte, d'autres organes d'étranglement (19, 20) étant éventuellement prévus, qui s'ouvrent lorsque la pression augmente encore.

5. Circuit de commande selon l'une des revendications 1 à 4, caractérisé en ce que les autres organes d'étranglement (17, 18, 19, 20), qui se ferment lorsque la pression monte et qui s'ouvrent lorsque la pression augmente encore, peuvent être rappelés grâce à l'action de ressorts.

6. Circuit de commande selon l'une des revendications 1, 4 ou 5, caractérisé en ce que l'organe de réglage disposé dans le passage (6) est conformé en étranglement (21), l'autre organe d'étranglement (10) réglable pouvant être contourné par une conduite de dérivation (22), dans laquelle une soupape de retenue (23) est disposée pour bloquer l'écoulement de la chambre de cylindre (5) contenant la tige de piston (3) vers la chambre de compensation (9). (Fig. 4).

7. Circuit de commande selon la revendication 1, caractérisé en ce que l'autre soupape de retenue (24) est disposée directement dans la conduite de liaison (25) entre la chambre de cylindre (5) contenant la tige de piston (3) et la chambre de compensation (9), l'autre organe d'étranglement (10) réglable étant situé dans une conduite de dérivation (26) contournant la soupape de retenue (24). (Fig. 5).
